# FASCICULE DE BREVET EUROPEEN

(11) **EP 1 052 942 B1**
(45) Date de publication et mention de la délivrance du brevet: **13.10.2004**
(21) Numéro de dépôt: 99904900.0
(22) Date de dépôt: 03.02.1999
(51) Int. Cl.: A61B 17/04

(54) **DISPOSITIF CHIRURGICAL D'ANCRAGE OSSEUX**
CHIRUGISCHE KNOCHENANKEREINRICHTUNG
SURGICAL BONE ANCHORING DEVICE

(30) Priorité: 06.02.1998 FR 9801415
(43) Date de publication de la demande: 22.11.2000
(73) Titulaire: Fumex, Laurent, 78730 Saint-Arnoult-en-Yvelines (FR)
(72) Inventeur: Fumex, Laurent, 78730 Saint-Arnoult-en-Yvelines (FR)
(74) Mandataire: L'Helgoualch, Jean
(86) Numéro de dépôt international: PCT/FR1999/000231
(87) Numéro de publication internationale: WO 1999/039644

(56) Documents cités:
- EP-A- 0 611 551
- EP-A- 0 861 633
- US-A- 5 403 348
- US-A- 5 423 860
- US-A- 5 545 178
- US-A- 5 584 695

## Description

La présente invention concerne un dispositif chirurgical d'ancrage osseux, et plus particulièrement un dispositif chirurgical permettant de réaliser simplement et efficacement un ancrage de fil de suture ou de fil chirurgical sur un support osseux, notamment en chirurgie orthopédique, traumatologique, gynécologique et carcinologique.

La rupture de tendons ou de ligaments est un accident qui peut survenir chez de nombreux individus de tous âges, actifs ou inactifs, à la suite de traumatismes ou d'efforts trop importants. Les techniques chirurgicales réparatrices couramment employées consistent à attacher le tendon, au moyen d'un fil de suture, à une vis, une cheville ou un piton fixé dans l'os voisin.

Pour cela, la technique classique consiste à forer dans l'os, soit un trou taraudé pour pouvoir y visser une vis ou un piton, soit un trou non taraudé recevant une cheville pouvant se bloquer dans l'os. La vis, le piton ou la cheville sert de moyen d'ancrage sur lequel on attache ensuite un fil de suture qui est utilisé pour rattacher le tendon qui a été arraché de son support osseux. Suivant d'autres techniques, après avoir foré un trou adapté à recevoir un piton d'ancrage, on fait passer le fil de suture dans le chas du piton d'ancrage, puis on introduit celui-ci dans le trou au moyen d'un appareillage spécial, et enfin on suture le tendon à fixer. Un exemple de vis d'ancrage pour fixation de tissus au moyen d'un fil de suture est décrit dans le brevet US-A-5.443.482.

Selon les cas, les interventions peuvent être effectuées soit en chirurgie à ciel ouvert, soit en chirurgie à ciel fermé au moyen de procédés coelioscopiques ou arthroscopiques. Des techniques chirurgicales utilisant des dispositifs de ce genre sont décrites par exemple par F. A. Barber et al., J. of Arthroscopy and Related Surgery, vol. 11, n° 1, p. 21-28 (1995).

Le brevet US-A-5.403.348 décrit un dispositif d'ancrage osseux comprenant une pièce cylindrique rigide traversée suivant son axe par un fil chirurgical, que l'on introduit dans un trou foré dans l'os, jusque dans la partie spongieuse. En agissant sur le fil, on modifie l'orientation de la pièce cylindrique de manière à la bloquer sous la couche superficielle solide de l'os cortical, en déformant la partie spongieuse.

Le brevet EP-A-611.551 décrit un dispositif d'ancrage pour fil de suture, comportant un rivet destiné à se fixer dans le support osseux, constitué par deux éléments : une pointe, et un manchon rigide dans lequel elle peut coulisser. Un autre dispositif d'ancrage est décrit dans le brevet US-A-5.584.695, comprenant un rivet à mémoire de forme, capable d'être introduit à froid dans le support osseux et de s'agrandir à chaud pour provoquer le blocage.

Ces dispositifs connus présentent divers inconvénients : ils sont très invasifs, non résorbables, et requièrent des techniques de mise en place souvent délicates à mettre en oeuvre; de plus certains dispositifs connus comprennent des produits contenant des matériaux allergisants, par exemple certains matériaux à mémoire de forme, et selon certains auteurs ces matériaux sont parfois cancérigènes. En outre, un grand nombre de vis, de chevilles ou de pitons, et d'accessoires de forage de l'os et de mise en place du moyen d'ancrage, doivent être disponibles dans des dimensions diverses, pour pouvoir répondre à toutes les situations.

La demande EP 861.633, publiée le 02/09/1998, au nom du demandeur, décrit un dispositif d'ancrage osseux comprenant un fil portant une gaine tubulaire pouvant se déformer après introduction dans un trou foré dans l'os ; à cet effet, la partie du fil portant la gaine est en forme de boucle fermée par un noeud coulant, et la gaine se déforme lorsque l'on tire sur les brins du noeud coulant.

La présente invention a pour objet un dispositif d'ancrage osseux nécessitant seulement le forage préalable d'un simple trou à paroi quelconque, sans requérir de taraudage, et permettant la fixation d'organes tels que des tendons et des ligaments, ou la suspension du col de l'utérus, au moyen d'un fil chirurgical ou d'un fil de suture sans qu'il soit nécessaire d'utiliser un moyen du type vis ou piton.

L'invention décrit également un matériel ancillaire spécialement adapté à la mise en place du dispositif d'ancrage précité.

Le dispositif d'ancrage conforme à la présente invention est utilisable en combinaison avec un trou foré dans le support osseux, et il comprend une gaine tubulaire déformable, associée à des moyens capables de la déformer entre une première position étirée de faible diamètre et une deuxième position repliée de diamètre plus important.

La longueur de la gaine tubulaire en position étirée doit être sensiblement supérieure au diamètre pour une efficacité maximale du dispositif. Plus précisément, la longueur de la gaine doit être égale à au moins 5 fois le diamètre, et de préférence au moins dix fois le diamètre extérieur de la gaine.

Suivant l'invention, les moyens capables de déformer la gaine sont constitués par un fil traversant la gaine tubulaire déformable suivant son axe et formant une boucle fermée, la gaine tubulaire se trouvant sur le fil, dans la partie en forme de boucle. Le diamètre intérieur de la gaine doit être suffisant pour contenir le fil sans opposer de force de frottement trop importante. A titre d'exemple, on peut utiliser une gaine tubulaire de longueur comprise entre 15 et 30 mm environ, de diamètre extérieur compris entre 1 et 2 mm, et de diamètre intérieur compris entre 0,6 et 1,2 mm.

Ainsi, dans sa forme préférentielle de réalisation, le dispositif d'ancrage osseux de l'invention comprend un fil comprenant une partie médiane destinée à être introduite dans le trou foré au préalable, présentant une forme de boucle fermée, portant sur la partie en boucle une enveloppe ou gaine tubulaire déformable pouvant coulisser sur le fil dans les limites de la boucle. La fermeture de la boucle peut être obtenue par simple croisement des deux brins du fil, de part et d'autre de la gaine déformable. La gaine tubulaire peut être déformée en boule par simple traction exercée sur au moins un brin du fil.

Suivant la présente invention, le fil utilisé est de préférence un fil chirurgical ou un fil de suture, résorbable ou non résorbable du type utilisé pour fixer ou rattacher des organes. On peut utiliser par exemple un fil en polyester tel que celui commercialisé sous la marque Ercylène®, ou un fil en polyamide tel que le Trynil®. Ainsi, le fil sert à la fois pour provoquer la déformation de la gaine tubulaire, mais aussi pour la fixation des organes.

L'enveloppe ou gaine tubulaire capable de coulisser sur le fil dans la boucle est d'une longueur totale inférieure ou égale à environ le double de la profondeur du perçage dans l'os, et d'un diamètre inférieur ou égal à celui du trou foré dans l'os. Ainsi, la boucle portant la gaine est entièrement introduite dans le trou foré dans l'os. Cette gaine peut avantageusement être réalisée en tous matériaux déformables, et présentant de préférence une certaine élasticité, ayant la propriété d'être implantables, résorbables ou non résorbables (par exemple un fil métallique ou plastique tressé, un tube en polyester ou en polyamide, ou un tube en silicone).

Selon les cas, la gaine peut être constituée par un seul élément ou plusieurs éléments.

Lors de la mise en place, après introduction de la gaine et du fil dans le trou foré dans l'os, lorsque l'on resserre la boucle en tirant sur au moins l'un des deux brins du fil, celui-ci vient s'appliquer contre la gaine et provoquer sa déformation, la faisant passer d'une première position étirée à une deuxième position repliée sur elle-même où sa section se trouve augmentée. Plus précisément, après avoir replié la gaine par son milieu de telle sorte que les brins du fil ressortent dans la même direction, et après l'avoir ainsi introduite dans le trou foré dans l'os, en exerçant une traction sur les brins du fil suivant des directions divergentes, on provoque le repliement de la gaine jusqu'à ce qu'elle se présente approximativement sous la forme d'une boule qui, en raison de son diamètre plus important que celui de la gaine, se trouve comprimée contre les parois du trou où la gaine a été introduite. En raison de cette pression le dispositif est alors retenu fermement dans le trou foré dans l'os.

Suivant une forme simple de réalisation, la gaine en forme de cylindre linéaire est enfilée sur le fil, puis la boucle est formée autour de la gaine.

Dans une autre forme simple de réalisation, la gaine peut être préformée en U et le fil pénètre dans la gaine par une de ses deux extrémités ouvertes, ressort par la deuxième extrémité et forme une boucle pour pénétrer à nouveau dans la gaine par la première extrémité pour sortir par la deuxième extrémité. Suivant une variante, la gaine peut comporter un orifice traversant sa paroi à proximité de chacune des deux extrémités, et le fil peut pénétrer dans la gaine puis en ressortir par l'un et l'autre de ces orifices. Dans cette dernière forme de réalisation, si les orifices sont disposés dissymétriquement par rapport au plan moyen de la boucle, la traction exercée sur les brins du fil pour provoquer la mise en compression de la gaine entraîne aussi un effet de basculement qui vient compléter et renforcer le frottement et le blocage contre les parois internes du trou foré dans l'os.

Ces orifices sont disposés symétriquement par rapport au milieu de la gaine déformable, à proximité des extrémités, ou dans sa partie centrale. Il est préférable de prévoir deux orifices disposés dans un même plan diamétral de la gaine.

Suivant une autre forme de réalisation de l'invention, la gaine se présente sous la forme d'un anneau torique présentant au moins un orifice traversant sa paroi pour le passage des deux brins du fil. Dans cette variante, le fil pénètre dans la gaine par l'orifice, forme une boucle complète en suivant l'intérieur de la gaine annulaire et ressort par le même orifice.

La gaine est de préférence renforcée dans la zone où pénètre le fil, c'est-à-dire sur la périphérie de chacune de ses extrémités, ou autour des orifices pratiqués dans sa paroi, le cas échéant. Ce renforcement peut être obtenu par exemple en prévoyant une surépaisseur de matière ou en fixant par collage ou soudage une bande présentant une résistance plus grande.

La gaine tubulaire peut être ouverte à ses deux extrémités ou, au contraire, être fermée. Dans ce cas, elle comporte au moins deux orifices dans sa paroi pour le passage des brins du fil. La paroi de la gaine tubulaire est de préférence traversée par au moins deux orifices pour le passage des brins du fil, disposés de telle sorte que la traction exercée sur les brins du fil provoque un mouvement de basculement de la gaine, favorisant son ancrage contre les parois du tour foré dans l'os.

L'ancillaire de mise en place du dispositif d'ancrage conforme à la présente invention comprend essentiellement une tige capable de porter le fil et sa gaine pour les introduire dans le trou foré dans l'os, ainsi qu'une pièce cylindrique fendue enveloppant le fil et la gaine et capable de coulisser sur la tige suivant une distance correspondant à la profondeur du trou.

Suivant une forme préférentielle de réalisation, le coulissement de la pièce cylindrique est guidé par une nervure formée sur la tige et coopérant avec la fente.

Suivant une autre caractéristique de la présente invention, l'ancillaire comprend un manche solidaire de la tige, et portant une poignée facilitant la manipulation.

La pièce cylindrique coulissante est réalisée en une matière compatible avec l'utilisation en milieu chirurgical.

L'utilisation de l'ancillaire pour la mise en place du dispositif d'ancrage de l'invention se fait facilement en introduisant la pointe de la tige de l'ancillaire, portant le fil en forme de boucle et sa gaine, dans le trou préalablement foré dans l'os, jusqu'à ce que la boucle soit totalement engagée dans le trou. Après avoir retiré la pointe hors du trou, tout en y laissant le fil et la gaine, il suffit alors d'exercer une traction sur un brin du fil, tout en freinant légèrement l'autre brin, pour provoquer le resserrement de la boucle à l'intérieur du trou dans l'os et la déformation de la gaine sur la boucle jusqu'à prendre la forme d'une boule dont le diamètre est supérieur à celui de la gaine tubulaire avant déformation. La traction étant prolongée jusqu'au blocage, la gaine déformée se trouve alors en compression contre les parois internes de la cavité.

On peut ensuite nouer les deux brins du fil, au bord du trou foré dans l'os, pour bloquer le dispositif et empêcher son desserrement, puis, si nécessaire, rattacher le tendon ou l'organe au moyen des brins du fil. De plus, lorsque la gaine est repliée en forme de boule, comme indiqué ci-dessus, l'agrandissement de son diamètre par rapport au diamètre initial de la gaine, et la forme plissée de sa surface augmentent le phénomène d'ancrage dans la partie spongieuse de l'os, et il en résulte un meilleur accrochage du dispositif de l'invention dans l'os.

Comme indiqué ci-dessus, le dispositif et l'ancillaire de pose suivant la présente invention sont tout particulièrement destinés à la chirurgie réparatrice de ligaments et tendons. L'invention peut aussi être utilisée en gynécologie, par exemple pour fixer l'utérus sur le sacrum par l'intermédiaire d'un ligament.

Le dispositif de l'invention convient encore à la chirurgie carcinologique, et dans cette application on utilise un fil qui peut contenir une substance radioactive telle que de l'iridium. Le fil est placé dans la tumeur, et en utilisant de préférence un fil non résorbable, ce dernier peut servir de repère pour l'ablation secondaire de la tumeur.

Les essais réalisés avec le dispositif d'ancrage suivant la présente invention, effectués dans des conditions expérimentales, ont permis de mettre en évidence d'excellentes propriétés de fixation et de résistance à la traction, comparables, voire supérieures, à celles des meilleurs dispositifs connus de la technique.

Les essais ont été effectués sur deux séries de dispositifs constitués par des gaines en tresse de polyester non résorbable à extrémités thermocollées de longueur égale à 20 mm pour la 1ère série, et 30 mm pour la 2ème série. Les gaines ont un diamètre extérieur de 1,6 mm et un diamètre intérieur de 1,0 mm. Un fil de suture en polyester de 0,5 mm de diamètre (USP 2) est glissé à l'intérieur de la gaine. Les extrémités du fil sont nouées afin de former une boucle.

Les gaines, portant le fil, sont introduites dans une série de trous de 2,3 mm de diamètre et 19 mm (gaines de 30 mm) ou 11 mm (gaines de 20 mm) de profondeur, forés dans un humérus frais humain. Les essais de résistance à l'arrachement dans l'axe du trou sont effectués au moyen d'une machine universelle de traction Adamel Lhomargy DY 34 équipée d'une cellule de forcé 1 kN étalonnée. Un barreau cylindrique solidaire de la traverse mobile de la machine est passé dans une boucle formée à l'autre extrémité de l'échantillon. La vitesse de déplacement de la traverse est de 5 mm/min. Les essais sont effectués jusqu'à obtenir l'arrachement du dispositif hors du support osseux ou la rupture du fil.

Dans tous les cas on observe une rupture du fil de suture sans extraction de la gaine hors du trou foré dans l'os. La force de résistance à l'arrachement varie entre 10,2 et 12 kg pour les échantillons des deux séries.

Les caractéristiques et avantages de la présente invention apparaîtront plus clairement dans les exemples suivants relatifs à des formes préférentielles de réalisation, en référence aux dessins annexés, qui représentent:
Figure 1 : une vue schématique d'un fil de fixation conforme à la présente invention, introduit dans une gaine en forme de U avant serrage et déformation.
Figure 2 : une vue d'une variante du dispositif de la Figure 1 où chaque brin du fil sort de la gaine tubulaire par un orifice à proximité de chaque extrémité.
Figure 3 : une vue du dispositif de la Figure 2 montrant la forme prise par la gaine tubulaire après une première traction sur les brins du fil.
Figure 4 : une vue d'une autre variante du dispositif de l'invention.
Figure 5 : une vue en perspective d'un matériel ancillaire permettant une mise en place semi-automatique d'un dispositif d'ancrage selon l'invention.

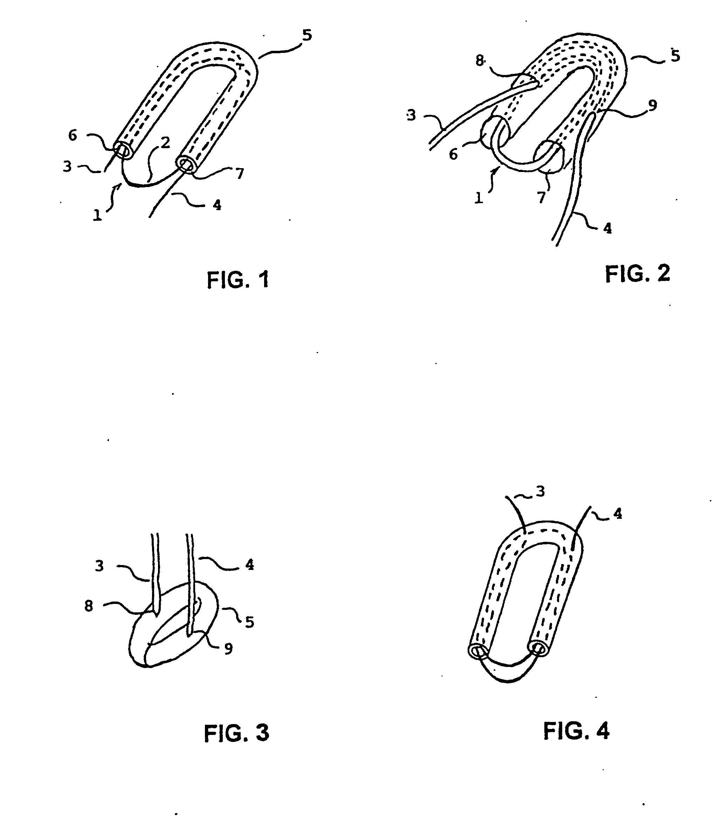

La Figure 1 représente le dispositif d'ancrage de la présente invention qui comprend un fil de suture (1) formant dans sa partie médiane une boucle fermée (2) susceptible d'être serrée par simple traction sur l'un des deux brins (3, 4) du fil (1).

Le fil (1) formant la boucle (2) pénètre dans la gaine (5) par son extrémité (6), ressort par l'autre extrémité (7), entre à nouveau par l'extrémité (6) et sort par l'extrémité (7) de manière à former une boucle complète dans la gaine. Celle-ci est en matière déformable et compressible, susceptible de glisser sur le fil. La traction sur les brins (3 et 4) du fil provoque une diminution de la longueur de la boucle (2) jusqu'à ce que sa longueur devienne égale à celle de la gaine (5). En continuant la traction sur les brins du fil (1), ou seulement sur l'un des brins en retenant l'autre, on provoque d'une part une compression de la gaine dont la surface forme des ondulations en raison de la compressibilité de la matière qui la constitue, et d'autre part le serrage de la boucle (2).

Les bords de la gaine, à chacune de ses extrémités (6) et (7), sont renforcés pour éviter qu'ils ne soient entaillés par le fil (1) lorsque celui-ci est serré. Ce renforcement est obtenu ici par une simple surépaisseur de matière.

La mise en place du dispositif s'effectue au moyen d'un matériel ancillaire tel que décrit ci-après. La méthode de mise en place consiste à replier par son milieu la boucle (2) portant la gaine (5) de telle sorte que les deux brins du fil sortent du même côté, comme le montre la Figure 1, et à l'introduire par son milieu dans le trou foré dans l'os, puis à serrer en exerçant une traction sur les brins (3 et/ou 4) du fil (1) suivant des directions divergentes. Il est préférable d'introduire la totalité de la boucle dans le trou, de telle sorte qu'aucune partie de la gaine ne dépasse hors du trou. De préférence, les bords (6) et (7) de la gaine sont enfoncés dans le trou sous la surface de l'os cortical.

Lorsque l'on serre la boucle (2) en tirant sur le ou les brins (3) et (4) du fil, on provoque son rétrécissement et la compression de la gaine flexible (5) à l'intérieur du trou. Puis en accentuant le serrage par traction sur le brin (3) du fil (1) on déforme la gaine (5) jusqu'à ce qu'elle prenne la forme d'une boule. Cette boule ne pourra ressortir par le trou par lequel on l'a introduite dans l'os car son diamètre est devenu nettement supérieur à celui du trou foré. De plus cette boule prend appui sur la face interne de l'os cortical ou dans l'os spongieux s'il est suffisamment dur.

Il suffit donc de choisir un fil de suture portant une gaine ayant une longueur et un diamètre permettant, par serrage de la boucle (2), la formation d'une boule qui prendra appui sur la face interne dure d'un os et dont le diamètre sera suffisamment important pour résister aux efforts qui lui sont imposés.

Sur la variante du dispositif représentée sur la Figure 2, au lieu de pénétrer dans la gaine (5) par les extrémités (6) et (7), le fil (1) traverse la gaine (6) par les orifices (8) et (9) prévus à proximité des extrémités de la gaine. Les deux orifices (8) et (9) sont situés de préférence du même côté par rapport à un plan diamétral de la gaine.

Cette forme de réalisation facilite le repliement de la gaine sur elle-même pour former un cercle comme représenté sur la Figure 3. Quand on continue à exercer une traction sur les brins du fil, ce cercle se déforme et la gaine forme des plis pour prendre sensiblement la forme d'une boule.

La Figure 4 montre une autre variante de l'invention où les brins (3) et (4) traversent la paroi de la gaine (5) par les orifices (8') et (9') disposés comme les orifices (8) et (9) du dispositif de la Figure 2, mais à proximité de la partie centrale de la gaine. Suivant cette variante, les brins du fil sont dirigés de préférence dans une direction opposée à celle des extrémités (6) et (7) de la gaine et celle-ci peut être introduite dans le trou foré dans l'os, par ses extrémités et non par son milieu.

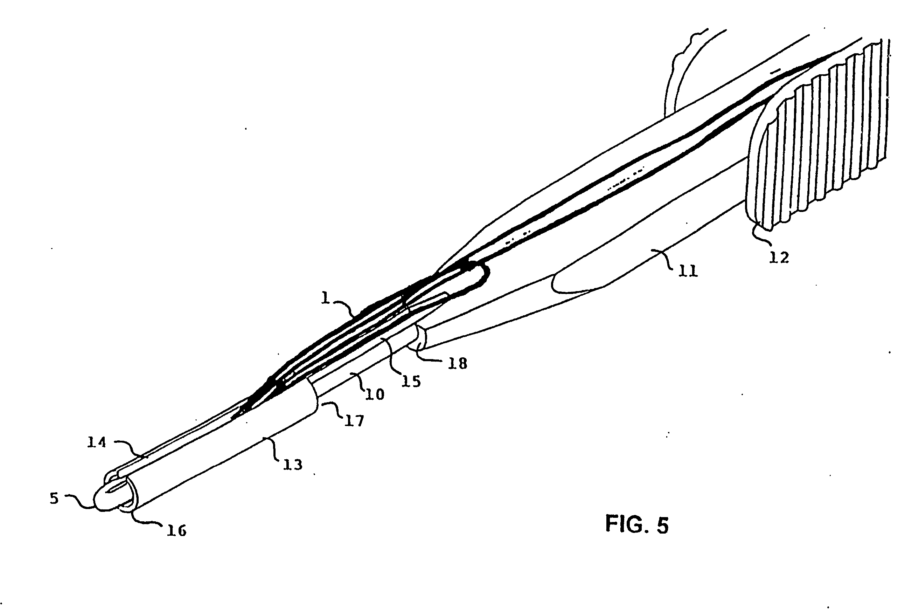

La pose du dispositif d'ancrage décrit ci-dessus s'effectue efficacement au moyen d'un matériel ancillaire conforme à l'invention, représenté sur la Figure 5.

Sur cette Figure est représenté un matériel ancillaire simple facilitant la mise en place du dispositif d'ancrage dans un trou préalablement foré dans un os.

Cet ancillaire comprend une tige (10) destinée à supporter la boucle du fil de suture (1) dans la gaine (5), par le milieu de sa boucle (2), pour assurer sa mise en place dans le trou foré dans l'os (non représenté). Comme le montre la Figure 4, le fil (1) et sa gaine (5) sont repliés par leur milieu sur l'extrémité libre de la tige (10). Cette tige (10) est fixée sur un manche (11) portant une poignée (12).

La tige (10), la gaine (5) et le fil (1) sont enserrés dans une pièce cylindrique (13) comportant une fente (14) sur toute sa longueur. Cette pièce cylindrique (13) peut coulisser sur la tige (10) et elle est guidée par la nervure (15) qui coopère avec la fente (14). Le fonctionnement est décrit ci-après.

Après forage du trou dans l'os au moyen d'un appareil usuel, on introduit la tige (10) portant le fil (1) formé en boucle et la gaine (5), dans le trou, de telle sorte que le bord distal (16) vienne en butée contre l'os, au bord du trou. On enfonce ensuite la tige (10), ce qui provoque le coulissement de l'élément cylindrique (13) suivant la nervure (15) jusqu'à ce que le bord proximal (17) de l'élément cylindrique (13) vienne en appui contre l'épaulement (18) situé à la base de la tige (10). La distance de coulissement est déterminée de manière à correspondre à la profondeur du trou foré dans l'os. Dans cette position, la gaine (5) est entièrement enfoncée dans le trou foré dans l'os. L'élément cylindrique (13) peut le cas échéant être remplacé par un élément plus long ou plus court selon la profondeur du forage.

On défait ensuite les brins du fil (1) fixés sur des plaquettes porte-fils (non représentée) solidaire du manche (11). On retire l'ancillaire du trou en agissant sur la poignée (11), puis on tire sur le brin (4) du fil (1) de manière à faire glisser le fil (1) dans la gaine (5) et à déformer cette dernière pour qu'elle forme une boule.

La mise en place du dispositif d'ancrage au moyen du matériel ancillaire de la Figure 5 s'effectue comme indiqué ci-dessus, de manière semi-automatique.

## Revendications

1. Dispositif chirurgical utilisable en chirurgie orthopédique, traumatologique, gynécologique et carcinologique, pour l'ancrage osseux dans un trou foré au préalable dans l'os, comprenant un fil (1) en forme de boucle fermée (2) portant une gaine tubulaire déformable et en sortant, capable de se déformer entre une première position étirée de faible section et une deuxième position repliée de plus forte section, par traction sur le fil (1), **caractérisé en ce que**
la gaine (5) est préformée en U et le fil (1) pénètre par une extrémité ouverte (6), ou par un orifice (8) à proximité, sort par l'autre extrémité (7), et forme ladite boucle fermée en pénétrant à nouveau dans la gaine (5) par la première extrémité (6) et en sortant par l'autre extrémité (7), ou par un orifice (9) à proximité de l'autre extrémité, ou bien
la gaine (5) est formée en anneau torique et le fil pénètre par un orifice, forme ladite boucle fermée en suivant l'intérieur de l'anneau torique, et ressort par le même orifice.

2. Dispositif selon la revendication 1, **caractérisé en ce que** la gaine (5) peut être déformée en boule par traction exercée sur au moins un brin (3, 4) du fil (1).

3. Dispositif selon l'une quelconque des revendications 1 et 2, **caractérisé en ce que** la longueur de la gaine (5) est inférieure ou égale au double de la profondeur du trou foré dans le support osseux.

4. Dispositif selon l'une quelconque des revendications précédentes, **caractérisé en ce que** le diamètre de la gaine (5) est inférieur ou égal au diamètre du trou.

5. Dispositif selon l'une quelconque des revendications précédentes, **caractérisé en ce que** la longueur de la gaine (5) est égale à au moins 5 fois le diamètre.

6. Dispositif selon la revendication 4, **caractérisé en ce que** la gaine (5) est constituée par un élément tubulaire fermé à ses deux extrémités et comportant au moins deux orifices (8, 9) pour le passage des brins du fil.

7. Dispositif selon la revendications 6, **caractérisé en ce que** la paroi de la gaine tubulaire est traversée par au moins deux orifices pour le passage des brins du fil, disposés de telle sorte que la traction exercée sur les brins du fil provoque un mouvement de basculement de la gaine.

## Claims

1. Surgical device which can be used in orthopedic surgery, traumatology, gynecology and cancerology, for bone anchoring in a hole bored beforehand in the bone, comprising a thread (1) in the form of a closed loop (2), bearing a deformable tubular sleeve and coming out therefrom, capable of deforming between a first stretched position of low cross section and a second folded position of greater cross section, by traction exerted on the thread (1), **characterized in that**
the sleeve (5) is U-shaped and the thread (1) enters through an open end (6), or through an orifice (8) close to it, goes out through the other end (7), and forms said closed loop by being once more introduced into the sleeve (5) through the first end (6), and coming out through the other end (7), or through an orifice (9) close to it, or
the sleeve is made up of a toric ring and the thread enters through an orifice, forms said closed loop following the inside of the toric ring, and comes out through the same orifice.

2. Device according to Claim 1, **characterized in that** the sleeve (5) can be deformed into a ball by traction exerted on at least one strand (3, 4) of the thread (1).

3. Device according to any of Claims 1 and 2, **characterized in that** the length of the sleeve (5) is less than or equal to twice the depth of the hole bored in the bone support.

4. Device according to any of the preceding claims, **characterized in that** the diameter of the sleeve (5) is less than or equal to the diameter of the hole.

5. Device according to any of the preceding claims, **characterized in that** the length of the sleeve (5) is equal to at least 5 times the diameter.

6. Device according to Claim 4, **characterized in that** the sleeve (5) is made up of a single tubular element which is open at both of its ends (6, 7).

7. Device according to Claim 6, **characterized in that** the wall of the tubular sleeve has at least two orifices extending through it for the passage of the strands of the thread, these orifices being arranged in such a way that the traction exerted on the strands of the thread causes a tilting movement of the sleeve.

## Patentansprüche

1. Chirurgische Vorrichtung, die in der orthopädischen, traumatologischen, gynäkologischen und karzinologischen Chirurgie zur Verankerung von Knochen in einem im Voraus im Knochen gebohrten Loch einsetzbar ist, umfassend einen Faden (1) in Form einer geschlossenen Schlinge (2), die eine verformbare, röhrenförmige Hülle (5) trägt, die durch Ziehen am Faden (1) zwischen einer ersten, ausgezogenen Position mit geringem Querschnitt und einer zweiten, zurückgezogenen Position mit größerem Querschnitt verformbar ist, **dadurch gekennzeichnet, dass**
die Hülle (5) U-förmig vorgeformt ist und der Faden (1) durch ein offenes Ende (6) oder eine in der Nähe befindliche Öffnung (8) eintritt und durch das andere Ende (7) austritt und durch neuerliches Eintreten in die Hülle (5) durch das erste Ende (6) und durch Austreten durch das andere Ende (7) oder durch eine in der Nähe des anderen Endes befindlichen Öffnung (9) die geschlossene Schlinge bildet, oder
die Hülle (5) in Form eines torischen Rings geformt ist und der Faden durch eine Öffnung eintritt, entlang dem Inneren des torischen Rings die geschlossene Schlinge bildet und aus derselben Öffnung wieder austritt.

2. Vorrichtung nach Anspruch 1, **dadurch gekennzeichnet, dass** die Hülle (5) durch Zugeinwirkung auf zumindest einen Teilfaden (3, 4) des Fadens (1) zu einer Kugel verformbar ist.

3. Vorrichtung nach einem der Ansprüche 1 oder 2, **dadurch gekennzeichnet, dass** die Länge der Hülle (5) kleiner als die oder gleich der doppelte(n) Tiefe des im Knochenträger gebohrten Lochs ist.

4. Vorrichtung nach einem der vorangegangenen Ansprüche, **dadurch gekennzeichnet, dass** der Durchmesser der Hülle (5) kleiner als der oder gleich dem Durchmesser des Lochs ist.

5. Vorrichtung nach einem der vorangegangenen Ansprüche, **dadurch gekennzeichnet, dass** die Länge der Hülle (5) zumindest fünfmal so groß wie der Durchmesser ist.

6. Vorrichtung nach Anspruch 4, **dadurch gekennzeichnet, dass** die Hülle (5) aus einem an seinen beiden Enden geschlossenen und mit mindestens zwei Öffnungen (8, 9) für den Durchtritt der Teilfäden des Fadens versehenden röhrenförmigen Element besteht.

7. Vorrichtung nach Anspruch 6, **dadurch gekennzeichnet, dass** die Wand der röhrenförmigen Hülle von zumindest zwei Öffnungen für den Durchtritt der Teilfäden des Fadens durchbohrt ist, die so angeordnet sind, dass die Zugeinwirkung auf die Teilfäden des Fadens eine Klappbewegung bewirkt.
